(19) Europäisches Patentamt
European Patent Office
Office européen des brevets

(11) EP 1 249 223 A1

(12) **DEMANDE DE BREVET EUROPEEN**

(43) Date de publication:
**16.10.2002 Bulletin 2002/42**

(21) Numéro de dépôt: **02290816.4**

(22) Date de dépôt: **02.04.2002**

(51) Int Cl.[7]: **A61K 7/02**, A61K 7/00, A41G 3/00, A41G 5/00, A63H 3/44

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **10.04.2001 FR 0104938**
**06.02.2002 FR 0201438**

(71) Demandeur: **L'OREAL**
**75008 Paris (FR)**

(72) Inventeurs:
- **Arnaud, Pascal**
**94240 L'Hay-Les-Roses (FR)**
- **Le Chaux, Laure**
**94240 L'Hay-Les-Roses (FR)**
- **Ferrari, Véronique**
**84700 Maisons-Alfort (FR)**
- **Lebre, Caroline**
**94320 Thiais (FR)**

(74) Mandataire: **Lhoste, Catherine**
**L'OREAL-DPI**
**6 rue Bertrand Sincholle**
**92585 Clichy Cédex (FR)**

(54) **Produit de maquillage bicouche, ses utilisations et kit de maquillage contenant ce produit**

(57) L'invention se rapporte à un produit cosmétique de maquillage contenant une première et une seconde compositions, la première composition comprenant, dans un milieu physiologiquement acceptable, des particules de polymère dispersées et stabilisées en surface par un stabilisant dans une phase organique liquide et un agent de coloration, et la seconde composition comprenant un second milieu physiologiquement acceptable.

L'invention se rapporte également à procédé de maquillage ainsi qu'à un kit de maquillage contenant ledit produit.

Ce dernier est en particulier un rouge à lèvres.

EP 1 249 223 A1

## Description

La présente invention se rapporte à un nouveau produit cosmétique de maquillage comprenant au moins deux compositions qui peuvent être appliquées successivement sur la peau aussi bien du visage que du corps humain, sur les paupières inférieures et supérieures des êtres humains, sur les lèvres et sur les phanères comme les ongles, les sourcils, les cils ou les cheveux, ainsi qu'un procédé de maquillage bicouche du visage et du corps humain.

Chaque composition peut être une poudre libre ou compactée, un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

Les compositions de maquillage de la peau ou des lèvres des êtres humains comme les fonds de teint ou les rouges à lèvres contiennent généralement des phases grasses telles que des cires et des huiles, des pigments et/ou des charges et éventuellement des additifs comme des actifs cosmétiques ou dermatologiques. Elles peuvent aussi contenir des composés dits "pâteux", de consistance souple, permettant d'obtenir des pâtes, colorées ou non, à appliquer au pinceau.

Les compositions connues ont tendance à migrer, c'est-à-dire à se propager au cours du temps à l'intérieur des plis des rides et des ridules de la peau qui entourent notamment les lèvres et les yeux, entraînant un effet inesthétique. Cette migration est souvent citée par les femmes comme un défaut majeur des rouges à lèvres et des fards à paupières classiques. Par "migration", on entend un débordement de la composition et en particulier de la couleur, hors du tracé initial du maquillage. En outre, ces compositions présentent une mauvaise tenue dans le temps et en particulier de la couleur. Cette mauvaise tenue se caractérise par une modification de la couleur (virage, palissement) généralement par suite d'une interaction avec le sébum et/ou la sueur sécrétés par la peau dans le cas de fond de teint et de fard ou d'une interaction avec la salive dans le cas des rouges à lèvres. Ceci oblige l'utilisateur à se remaquiller très souvent, ce qui peut constituer une perte de temps.

Depuis plusieurs années la recherche cosmétique s'intéresse aux compositions de maquillage pour les lèvres et la peau dites « sans transfert » c'est à dire aux compositions qui présentent l'avantage de former un dépôt qui ne se dépose pas, au moins en partie, sur les supports avec lesquels elles sont mises en contact (verre, vêtements, cigarette, tissus etc...).

Les compositions sans transfert connues sont généralement à base de résines de silicone et d'huiles de silicone volatiles et, bien que présentant des propriétés de tenue améliorées, ont l'inconvénient de laisser sur la peau et les lèvres, après évaporation des huiles de silicone volatiles, un film qui devient inconfortable au cours du temps (sensation de dessèchement et de tiraillement), écartant un certain nombre de femmes de ce type de rouge à lèvres.

En outre, ces compositions à base d'huiles volatiles de silicone et de résines siliconées conduisent à des films colorés mats. Or, les femmes sont aujourd'hui à la recherche de produits, notamment de coloration des lèvres ou des paupières, brillants, tout en étant de bonne tenue, et sans transfert.

Pour remédier à ces inconvénients, le demandeur a envisagé la fabrication de compositions de maquillage contenant des particules de polymère dispersées et stabilisées en surface par un stabilisant dans une phase grasse liquide, telles que décrites dans le document EP-A-0 930 060. Toutefois, ces compositions ne permettent pas d'obtenir un maquillage réellement brillant, toujours recherché par les consommatrices.

La société Kose a par ailleurs proposé dans sa demande de brevet JP-A-0 5 221 829 l'utilisation d'un gel à base de matières perfluorées, que l'on applique sur un film de rouge à lèvres de manière à éviter son transfert sur d'autres surfaces, le gel étant incompatible avec le film de rouge à lèvres.

Bien que l'utilisation d'huiles perfluorées assure une incompatibilité entre le gel et le film de rouge à lèvre, et donc des propriétés de tenue et de non transfert, les formulations de ce type présentent l'inconvénient d'une mauvaise cosméticité, le film de rouge à lèvres devenant huileux et susceptible de migrer, ce qui est inacceptable pour les consommatrices.

On peut aussi citer la demande de brevet WO-A-97/17057 de la société Procter et Gamble qui décrit une méthode pour augmenter les propriétés de tenue et de non transfert consistant à appliquer deux compositions l'une sur l'autre. Ces deux compositions satisfont aux critères physico-chimiques suivants :

- paramètres de solubilité global d'Hildebrand inférieur à 8,5$(cal/cm^3)^{½}$. pour la composition appliquée en premier
- présence d'huile dont le coefficient de partage calculé ClogP est au moins égal à 13 pour la surcouche.

Cependant, cette sélection de composition n'exclut pas la possibilité d'avoir dans les deux compositions les mêmes constituants.

En effet les triglycérides, en particulier l'huile d'amande douce et l'huile d'olive, cités comme vérifiant le critères du coefficient de partage, ont aussi des paramètres de solubilité d'Hildebrand inférieurs à 8,5 $(cal/cm^3)^{½}$ (Vaughan C.D « Solubility effects in product, package, penetration and préservation », Cosmetics and Toiletries, vol. 103, p 47-69,

1988):

Huile d'amande douce : 6,81 $(cal/cm^3)^{1/2}$.
Huile d'olive 7,87 $(cal/cm^3)^{1/2}$.

De ce fait, il demeure une certaine compatibilité entre les deux couches, ce qui ne permet pas d'avoir des propriétés de tenue et de non transfert totalement satisfaisantes.

Enfin, le brevet US-A-6 001 374 de Nichols propose un système de maquillage multicouche consistant à utiliser une composition contenant une résine soluble dans l'alcool et insoluble dans l'eau, que l'on peut appliquer comme couche de base ou en surcouche, et présentant l'avantage de ne pas tâcher un support mis au contact du maquillage et de résister à l'eau et aux frottements, tout en ayant un certain brillant.

Cependant, cette composition contient un alcool hydrosoluble, en particulier l'éthanol, composé qui présente un caractère irritant, desséchant pour la peau et plus spécialement pour les lèvres, et qui est particulièrement inconfortable lorsque la peau ou les lèvres sont abîmées. De plus, cette composition exige l'utilisation d'un démaquillant particulier, ce qui est peu pratique.

La présente invention à pour but de proposer un produit de maquillage qui réunisse à la fois les propriétés de « sans transfert », de non migration, de tenue, de confort, de non dessèchement et de brillance, résultat qui n'a pas été obtenu de façon satisfaisante jusqu'ici.

Le demandeur a constaté de manière surprenante qu'en associant une première composition comprenant un milieu physiologiquement acceptable contenant des particules de polymère dispersées et stabilisées en surface par un stabilisant dans une phase organique liquide et un agent de coloration et une seconde composition comprenant un milieu physiologiquement acceptable, on obtient un maquillage bicouche brillant qui ne migre pas, ne transfère pas, tout en étant confortable à l'application et dans le temps (non desséchant, pas de tiraillement).

En particulier, le produit de l'invention permet l'obtention de dépôts continus non collants, de bonne couvrance, ayant un aspect brillant, adapté au désir de la consommatrice, ne migrant pas, ne transférant pas et de bonne tenue, non huileux, ne desséchant pas la peau ou les lèvres sur lesquelles il est appliqué, aussi bien lors de l'application qu'au cours du temps. Il présente, en outre de bonnes propriétés de stabilité et permet ainsi un maquillage homogène et esthétique.

On a de plus constaté que les compositions utilisées dans le procédé selon l'invention présentent des qualités d'étalement et d'adhésion sur la peau, les lèvres, les cils ou les muqueuses, particulièrement intéressantes, ainsi qu'un toucher onctueux et agréable. Les compositions ont, en outre, l'avantage de se démaquiller facilement notamment avec un démaquillant classique.

Ces propriétés de tenue, de non transfert et de non migration, alliées à l'aspect brillant et confortable, non gras, en font un produit particulièrement approprié pour réaliser des produits de maquillage des lèvres tels que rouges à lèvres, brillants à lèvres ou des yeux tels que mascara, eye-liners, fards à paupières.

L'objet de la présente invention est donc un produit cosmétique de maquillage contenant une première et une seconde compositions, la première composition comprenant, dans un premier milieu physiologiquement acceptable, des particules de polymère dispersées et stabilisées en surface par un stabilisant dans une phase organique liquide et un agent de coloration, et la seconde composition comprenant un second milieu physiologiquement acceptable.

Par produit de maquillage, on entend un produit contenant un agent colorant permettant le dépôt d'une couleur sur une matière kératinique (la peau, les lèvres ou les phanères) de personne humaine par l'application sur la matière kératinique de produits tels que des rouges à lèvres, des fards, des eye-liners, des fonds de teint ou des autobronzants, des produits de maquillage semi permanent (tatouage) ;

Le produit selon l'invention comprend deux (ou plusieurs) compositions physiologiquement acceptables conditionnées séparément ou ensemble dans un même article de conditionnement ou dans deux (ou plusieurs) articles de conditionnement séparés ou distincts.
De préférence, ces compositions sont conditionnées séparément et avantageusement, dans des articles de conditionnement séparés ou distincts.

L'objet de la présente invention est donc en particulier un produit cosmétique de maquillage se présentant sous la forme d'un fond de teint, d'un fard à joues ou à paupières, d'un rouge à lèvres, d'un produit ayant notamment des propriétés de soin, d'un eye-liner, d'un produit anti-cernes, ou d'un produit de maquillage du corps (du type tatouage).

L'invention a aussi pour objet un kit de maquillage contenant un produit cosmétique de maquillage tel que défini précédemment, dans lequel les différentes compositions sont conditionnées séparément et sont accompagnées de moyens d'application appropriés. Ces moyens peuvent être des pinceaux, des brosses, des stylos, des crayons, des feutres, des plumes, des éponges et /ou des mousses. De préférence, on utilise des feutres.

La première composition du produit selon l'invention peut constituer une couche de base appliquée sur la

matière kératinique et la seconde composition une couche de dessus ou « top coat ». Il est toutefois possible d'appliquer sous la première couche une sous couche ayant la constitution ou non de la seconde couche.
Il est aussi possible de déposer une surcouche sur la seconde couche ayant une constitution identique ou non à celle de la première couche. De préférence, le maquillage obtenu est un maquillage bicouche.

En particulier la couche de base est un fond de teint, un fard, un rouge à lèvres, un brillant à lèvres, un eye liner, un produit de maquillage du corps, et la couche du dessus (ou « top coat ») un produit de soin ou de protection.

L'invention se rapporte aussi à un procédé de maquillage de la peau et/ou des lèvres et/ou des phanères consistant à appliquer sur la peau et/ou les lèvres et/ou les phanères un produit cosmétique de maquillage tel que défini précédemment.

L'invention a encore pour objet un procédé de maquillage de la peau et/ou des lèvres et/ou des phanères d'être humain, consistant à appliquer sur la peau, les lèvres et/ou les phanères une première couche d'une première composition comprenant, dans un milieu physiologiquement acceptable, des particules de polymère dispersées et stabilisées en surface par un stabilisant dans une phase organique liquide et un agent de coloration, puis à appliquer, sur tout ou partie de la première couche, une seconde couche d'une seconde composition comprenant un milieu physiologiquement acceptable.

Plus précisément, le procédé selon l'invention consiste à appliquer sur la peau, les lèvres et/ou les phanères d'être humain une première couche d'une première composition comprenant, dans un premier milieu physiologiquement acceptable, des particules de polymère dispersées et stabilisées en surface par un stabilisant dans une phase organique liquide et un agent de coloration, à laisser sécher ladite première couche, puis à appliquer, sur tout ou partie de la première couche, une seconde couche d'une seconde composition comprenant un second milieu physiologiquement acceptable.

Ce maquillage bicouche peut être adapté à tous les produits de maquillage de la peau aussi bien du visage que du scalp et du corps d'êtres humains, des muqueuses comme les lèvres, de l'intérieur des paupières inférieures, et des phanères comme les ongles, les cils, les cheveux, les sourcils, voire les poils. La seconde couche peut former des motifs et peut être appliquée avec un stylo, crayon ou tout autre instrument (éponge, doigt, pinceau, brosse, plume etc.). Ce maquillage peut aussi être appliqué sur les accessoires de maquillage comme les faux ongles, faux cils, perruques ou encore des pastilles ou des patchs adhérents sur la peau ou les lèvres (du type mouches).

L'invention a également pout objet une composition cosmétique pour la mise en oeuvre du procédé de maquillage décrit ci-dessus. Cette composition comprend, dans un milieu physiologiquement acceptable, des particules de polymère dispersées et stabilisées en surface par un stabilisant dans une phase organique liquide, un agent de coloration et un agent rhéologique choisi parmi les copolymères d'oléfines à cristallisation controlée et leurs mélanges. De préférence, l'agent rhéologique est un copolymère éthylène/octène.

L'invention a aussi pour objet un support maquillé comprenant une première couche d'une première composition comprenant, dans un premier milieu physiologiquement acceptable, des particules de polymère dispersées et stabilisées en surface par un stabilisant dans une phase organique liquide et une seconde couche d'une seconde composition déposée sur tout ou partie de la première couche, comprenant un second milieu physiologiquement acceptable.

Ce support peut être en particulier un postiche tel qu'une perruque, des faux ongles, des faux cils, ou encore des patchs adhérents sur la peau ou les lèvres (du type mouches).

L'invention se rapporte également à l'utilisation cosmétique du produit cosmétique de maquillage défini ci-dessus pour améliorer les propriétés de confort et/ou de brillance et/ou de transfert et/ou de migration et /ou de tenue du maquillage sur la peau et/ou les lèvres et/ou les phanères.

L'invention a enfin pour objet l'utilisation d'un produit cosmétique de maquillage contenant une première et une seconde compositions, la première composition comprenant, dans un milieu physiologiquement acceptable, des particules de polymère dispersées et stabilisées en surface par un stabilisant dans une phase organique liquide et un agent de coloration, et la seconde composition comprenant un milieu physiologiquement acceptable, pour conférer à la peau et/ou les lèvres et/ou les phanères un maquillage confortable, brillant, non transfert et/ou non migrant et /ou de bonne tenue.

## Première composition

La première composition selon l'invention comprend donc, dans un premier milieu physiologiquement acceptable, des particules de polymère dispersées et stabilisées en surface par un stabilisant dans une phase organique liquide (ci-après appelées « dispersion de polymère ») et un agent de coloration.

Par «milieu physiologiquement acceptable », on entend un milieu non toxique et susceptible d'être appliqué sur la peau, les phanères ou les lèvres du visage d'êtres humains.

Par « cosmétiquement acceptable », on entend au sens de l'invention une composition d'aspect, d'odeur, de goût et de toucher agréables.

***Polymère en dispersion***

Selon l'invention le polymère est un solide insoluble dans la phase organique liquide de la première composition même à sa température de ramollissement, à l'inverse d'une cire même d'origine polymérique qui est elle soluble dans la phase organique liquide (ou phase grasse) à sa température de fusion. Il permet, en outre, la formation d'un dépôt notamment filmogène continu et homogène et/ou est caractérisé par l'enchevêtrement des chaînes polymériques. Avec une cire même obtenue par polymérisation on obtient, après fusion dans la phase organique liquide, une recristallisation. Cette recristallisation est en particulier responsable de la perte de la brillance de la composition.

Pour avoir des propriétés de sans transfert optimales, on choisit la quantité de polymère en fonction de la quantité de matières colorantes et/ou d'actifs et/ou d'huiles, contenue dans la première composition. En pratique, la quantité de polymère peut être supérieure à 2 % en poids (en matière active), par rapport au poids total de la composition.

Un avantage de l'utilisation d'une dispersion de particules de polymère dans une composition de l'invention est que ces particules restent à l'état de particules élémentaires, sans former d'agglomérats, dans la phase grasse. Un autre avantage de la dispersion de polymère est la possibilité d'obtenir des compositions très fluides (de l'ordre de 130 centipoises), même en présence d'un taux élevé de polymère.

Encore un autre avantage d'une telle dispersion de polymère est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau, les lèvres ou les phanères.

Encore un avantage de la dispersion de polymère de la composition de l'invention est la possibilité de faire varier la température de transition vitreuse (Tg) du polymère ou du système polymérique (polymère plus additif du type plastifiant), et de passer ainsi d'un polymère dur à un polymère plus ou moins mou, permettant de régler les propriétés mécaniques de la composition en fonction de l'application envisagée et en particulier du film déposé.

La première composition du produit selon l'invention comprend donc avantageusement au moins une dispersion stable de particules de polymère généralement sphériques d'un ou plusieurs polymères, dans une phase organique liquide physiologiquement acceptable. Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500nm. Il est toutefois possible d'obtenir des tailles de particules de polymère allant jusqu'à 1µm.

De préférence, les particules de polymères en dispersion sont insolubles dans les alcools hydrosolubles tels que, par exemple, l'éthanol.

Les polymères en dispersion utilisables dans la première composition de l'invention ont de préférence un poids moléculaire de l'ordre de 2000 à 10 000 000 et une Tg de -100°C à 300°C et mieux de -50° à 100°C, de préférence de -10°C à 50°C.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère. On peut aussi utiliser des agents de coalescence afin d'aider la polymère à former un dépôt continu et homogène.
Les agents de coalescence ou plastifiants utilisables dans l'invention sont notamment ceux cités dans le document FR-A-2782 917.

Il est possible d'utiliser des polymères filmifiables, de préférence ayant une Tg basse, inférieure ou égale à la température de la peau et notamment inférieure ou égale à 40°C.

De préférence le polymère utilisé est filmifiable, c'est-à-dire apte à former seul ou en association avec un agent plastifiant, un film isolable. Il est toutefois possible d'utiliser un polymère non filmifiable.

Par « polymère non filmifiable », on entend un polymère non capable de former, seul, un film isolable. Ce polymère permet, en association avec un composé non volatil du type huile, de former un dépôt continu et homogène sur la peau et/ou les lèvres.

Parmi les polymères filmifiables, on peut citer des homopolymères ou des copolymères radicalaires, acryliques ou vinyliques, de préférence ayant une Tg inférieure ou égale à 40°C et notamment allant de - 10° à 30°C, utilisés seul ou en mélange.

Parmi les polymères non filmifiables, on peut citer des homopolymères ou copolymères radicalaires, vinyliques ou acryliques, éventuellement réticulés, ayant de préférence une Tg supérieure à 40°C et notamment allant de 45° à 150°C, utilisés seul ou en mélange.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des

polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymères d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styréniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le ou les polymères en dispersion dans la phase liquide organique peuvent représenter en matière sèche de 2 à 40% du poids de la composition, de préférence de 5 à 30 % et mieux de 8 à 20%. Lorsque les particules de polymère en dispersion sont stabilisées en surface par un stabilisant solide à température ambiante, la quantité en matière sèche de la dispersion représente la quantité totale de polymère + stabilisant, sachant que la quantité de polymère ne peut être inférieure à 2%.

***Stabilisant***

Les particules de polymère en milieu organique sont stabilisées en surface, au fur et à mesure de la polymérisation, grâce à un stabilisant qui peut être un polymère séquencé, un polymère greffé, et/ou un polymère statistique,

seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly-12-(hydroxystéarique).

Ainsi on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu le milieu de synthèse puis la phase organique de la première composition contient une phase siliconée.

On peut aussi utiliser des copolymères blocs greffés ou séquencés comprenant au moins un bloc de type polyorganosiloxane et au moins un bloc d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "Dow Corning 3225C" par la société Dow Corning, les lauryl méthicones tels que ceux vendu sous la dénomination "Dow Corning Q2-5200 par la société "Dow Corning".

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile) ; OS129880, OS129881 et OS84383 de chez Lubrizol (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le milieu de synthèse organique envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le milieu de synthèse est apolaire, il est préférable de choisir en tant que stabilisant, un polymère

apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque la phase organique liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,
- (c) les copolymères blocs greffés ou séquencés comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

***<u>Phase organique liquide de la première composition</u>***

Par « phase organique liquide » selon l'invention, on entend tout milieu non aqueux liquide à température ambiante (25°C) et pression atmosphérique (760 mm de Hg), composé d'un ou plusieurs corps gras liquides à température ambiante, appelés aussi huiles. Cette phase organique liquide est macroscopiquement homogène (à savoir homogène à l'oeil nu). Cette phase organique peut contenir une phase organique liquide volatile et/ou une phase organique non volatile.

Par « phase organique non volatile », on entend tout milieu susceptible de rester sur la peau ou les lèvres pendant plusieurs heures. Une phase organique liquide non volatile a en particulier une pression de vapeur à température ambiante et pression atmosphérique, non nulle inférieure à 0,02 mm de Hg (2,66 Pa) et mieux inférieure à $10^{-3}$ mm de Hg (0,13 Pa).

Par « phase organique volatile », on entend tout milieu non aqueux susceptible de s'évaporer de la peau ou des lèvres, en moins d'une heure à température ambiante et pression atmosphérique. Cette phase volatile comporte notamment des huiles ayant une pression de vapeur, à température ambiante (25°C) et pression atmosphérique (760mm Hg) allant de 0,02 à 300 mm de Hg (2,66 Pa à 40 000 Pa) et de préférence de 0,05 à 300 mm de Hg (6,65 Pa à 40 000 Pa).

Avantageusement, la phase organique volatile contient une ou plusieurs huiles organiques volatiles dont le point éclair va de 30°C à 102°C.

Les corps gras liquides ou huiles composant la phase liquide organique sont choisis parmi les huiles d'origine minérale, animale, végétale ou synthétique, carbonées, hydrocarbonées, fluorées et/ou siliconées, seules ou en mélange dans la mesure où elles forment un mélange homogène et stable macroscopiquement et où elles sont appropriées à l'utilisation envisagée.

Par "huile hydrocarbonée", on entend des huiles contenant majoritairement des atomes de carbone et des atomes d'hydrogène et en particulier des chaînes alkyle ou alcényle comme les alcanes ou alcènes mais aussi les huiles à chaîne alkyle ou alcényle comportant un ou plusieurs groupements alcool, éther, ester ou acide carboxylique.

La phase organique liquide totale de la première composition peut représenter de 5 % à 98% du poids total de la composition et de préférence de 20 à 85 %. Avantageusement, elle représente au moins 30 % du poids total de la composition.

Comme huiles non volatiles utilisables dans l'invention, on peut citer les huiles hydrocarbonées d'origine minérale ou synthétique telles les hydrocarbures linéaires ou ramifiés comme l'huile de paraffine ou ses dérivés, l'huile de vaseline, les polydécènes, le polyisobutène hydrogéné tel que le Parléam commercialisé par la société Nippon Oil Fats, le squalane d'origine synthétique ou végétale ; les huiles d'origine animale comme l'huile de vison, de tortue, le perhydrosqualène ; les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, comme l'huile d'amande douce, de calophyllum, de palme, de pépins de raisin, de sésame, d'arara, de colza, de tournesol, de coton, d'abricot, de ricin, de luzerne, de courge, de

cassis, de macadamia, dé rosier muscat, de noisette, d'avocat, de jojoba, d'olive ou de germes de céréales (maïs, blé, orge, seigle) ; des esters d'acide gras et notamment d'acide lanolique, d'acide oléique, d'acide laurique, d'acide stéarique ; les esters de synthèse de formule $R_1COOR_2$ dans laquelle $R_1$ représente le reste d'un acide gras supérieur linéaire ou ramifié comportant de 7 à 40 atomes de carbone et $R_2$ représente une chaîne hydrocarbonée ramifiée contenant de 3 à 40 atomes de carbone, comme par exemple l'huile de Purcellin (octanoate de cétostéaryle), l'isononanoate d'isononyle, le benzoate d'alcool en $C_{12}$ à $C_{15}$, le palmitate d'éthyl 2-hexyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthyl-hexyle, le laurate de 2-hexyl-décyle, le palmitate de 2-octyl-décyle, le myristate de 2-octyl-dodécyle, le succinate de 2-diéthyl-hexyle, le malate de diisostéaryle, le triisostéarate de glycérine ou de diglycérine ; les esters hydroxylés comme le lactate d'isostéaryle ; les esters du pentaérythritol ; les acides gras supérieurs en $C_8$-$C_{26}$ tels que l'acide oléique, l'acide linoléique, l'acide linolénique ou l'acide isostéarique ; les alcools gras supérieurs en $C_8$-$C_{26}$ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alcoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou amine ; les polysiloxanes modifiés par des acides gras, des alcools gras ou des polyoxyalkylènes comme les diméthicones copolyols ou les alkylméthicones copolyols ; les silicones fluorées liquides ; ou encore les triglycérides des acides caprylique/caprique comme ceux vendus par la société Stearineries Dubois ou ceux vendus sous les dénominations Miglyol 810, 812 et 818 par la société Dynamit Nobel ; et leurs mélanges.

Avantageusement, la phase organique liquide peut contenir une ou plusieurs huiles organiques volatiles à température ambiante comme des huiles cosmétiques volatiles. Ces huiles sont favorables à l'obtention d'un dépôt de bonne tenue et non transfert. Après évaporation de ces huiles, on obtient un dépôt filmogène souple, non collant sur la peau ou les lèvres. Ces huiles volatiles facilitent, en outre, l'application de la composition sur la peau, les lèvres et les phanères. Elles peuvent être hydrocarbonés, siliconés et/ou fluorés et comporter éventuellement des groupements alkyle ou alkoxy, pendants ou en bout de chaîne siliconée.

Comme huile organique volatile utilisable dans l'invention, on peut citer les huiles de silicones linéaires ou cycliques ayant une viscosité à température ambiante inférieure à 8 $mm^2/s$ et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Comme autre huile volatile utilisable dans l'invention, on peut citer les huiles volatiles hydrocarbonées ayant de 8 à 16 atomes de carbone et leurs mélanges et notamment les alcanes ramifiés en $C_8$-$C_{16}$ comme les isoalcanes (appelées aussi isoparaffines) en $C_8$-$C_{16}$, l'isododécane, l'isodécane, l'isohexadécane et par exemple les huiles vendues sous les noms commerciaux d'Isopars' ou de Permethyls', les esters ramifiés en $C_8$-$C_{16}$ comme le néo pentanoate d'iso-hexyle et leurs mélanges.

Avantageusement, la ou les huiles organiques volatiles représentent de 20 à 90%, de préférence de 30 à 80% et mieux encore, de 40 à 70% du poids total de la première composition.

Lorsque la phase organique liquide de la première composition contient une huile volatile, cette première composition forme de préférence la couche de base du maquillage bicouche.

La dispersion de polymère en phase organique liquide peut être fabriquée comme décrit dans le document EP-A-0 749747. La polymérisation peut être effectuée en dispersion, c'est-à-dire par précipitation du polymère en cours de formation, avec protection des particules formées par un stabilisant. Dans ce cas, on prépare un mélange comprenant les monomères initiaux ainsi qu'un amorceur radicalaire, mélange qui est ensuite dissous dans un milieu appelé, dans la suite de la présente description, « milieu de synthèse organique». Lorsque la phase organique est une huile non volatile, on peut effectuer la polymérisation dans un milieu organique apolaire (milieu de synthèse) puis ajouter l'huile non volatile (qui doit être miscible avec ledit milieu de synthèse) et distiller sélectivement le milieu de synthèse.

On choisit donc un milieu de synthèse tel que les monomères initiaux et l'amorceur radicalaire y soient solubles, et les particules de polymère obtenues y soient insolubles afin qu'elles y précipitent lors de leur formation. En particulier, on peut choisir le milieu de synthèse parmi les alcanes tels que l'heptane, l'isododécane ou le cyclohexane.

Lorsque la phase organique liquide de la première composition contient une huile volatile, on peut directement effectuer la polymérisation dans ladite huile qui joue donc également le rôle de milieu de synthèse. Les monomères

doivent également y être solubles, ainsi que l'amorceur radicalaire, et le polymère obtenu doit y être insoluble.

Les monomères sont de préférence présents dans le milieu de synthèse, avant polymérisation, à raison de 5-20% en poids du mélange réactionnel. La totalité des monomères peut être présente dans le milieu avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

***Agent rhéologique***

Avantageusement, la première composition contient un ou plusieurs agent rhéologique structurant et/ou gélifiant de son milieu physiologiquement acceptable.

Ce ou ces agents rhéologiques sont des agents capables d'épaissir et/ou gélifier la composition. Il peuvent être présents en une quantité efficace pour augmenter la viscosité de la composition jusqu'à l'obtention d'un gel solide, à savoir un produit ne s'écoulant pas sous son propre poids, voire même en stick.

L'agent rhéologique représente notamment de 0,1 à 50 % du poids total de la première composition et mieux de 1 à 25 %.

Cet agent rhéologique est avantageusement choisi parmi les gélifiants lipophiles, les cires, les charges et leurs mélanges.

***Gélifiant lipophile***

Selon un mode de réalisation de l'invention, la première composition peut comprendre, comme agent rhéologique, un agent gélifiant de la phase organique.

Comme gélifiant de phase organique, on peut citer les argiles éventuellement modifiées comme les hectorites modifiées par un chlorure d'ammonium d'acide gras en $C_{10}$ à $C_{22}$, comme l'hectorite modifiée par du chlorure de distéaryl di-méthyl ammonium ; la silice pyrogénée éventuellement traitée hydrophobe en surface dont la taille des particules est inférieure à 1µm ; les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; les galactommananes comportant un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et plus particulièrement la guar éthylée ayant un degré de substitution de 2 à 3 telle que celle vendue par la société Aqualon sous le nom N-Hance-AG ; l'éthylcellulose comme celles vendues sous le nom d'Ethocel par Dow Chemical ; les gommes notamment siliconées comme les PDMS ayant une viscosité > 100 000 centistokes et leurs mélanges.

L'agent rhéologique peut aussi être choisi parmi les homopolymères ou copolymères de l'éthylène dont la masse moléculaire en poids est comprise entre 300 et 500000, et mieux entre 500 et 100000.

De préférence, l'agent rhéologique est choisi parmi les copolymères d'oléfines à cristallisation controlée tels que décrits dans la demande EP-A-1 034 776 du demandeur comme par exemple le copolymère d'éthylène/octène vendu sous la référence Engage 8400 par la société Dupont de Nemours. On obtient en effet avec ce type d'agent gélifiant un film de première composition, et par conséquent un maquillage final, qui présentent des propriétés de tenue et de non transfert particulièrement intéressantes.

Ce ou ces agents rhéologiques sont utilisés par exemple à des concentrations de 0,5 à 20 % et mieux de 1 à 10% du poids total de la première composition.

***Cire***

L'agent de rhéologie peut en outre comprendre une cire choisi parmi les cires solides à température ambiante, telles que les cires hydrocarbonées comme la cire d'abeilles éventuellement modifiée, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène ou de copolymère d'éthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic Press (1977), pages 21-32. De préférence, les cires utilisées ont une température de fusion au moins égale à 45°C.

Les cires peuvent être présentes à raison de 0,1 à 50% en poids dans la première composition et mieux de 3 à 25%, en vue de ne pas trop diminuer la brillance de cette composition et du film déposé sur les lèvres et/ou la peau.

***Charge***

L'agent rhéologique peut, de plus, comprendre une charge. Par « charge », on entend toute particule incolore ou blanche choisie parmi les charges minérales ou organiques, lamellaires, sphériques ou oblongues, chimiquement inerte dans la première composition. On peut citer le talc, le mica, la silice, le kaolin, les poudres de polyamide comme le Nylon® (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile telles que l'Expancel® (Nobel Industrie), les particules de polymère acrylique, notamment de copolymère d'acide acrylique comme le Polytrap® (Dow Corning) et les microbilles de résine de silicone (Tospearls® de Toshiba, par exemple), le carbonate de calcium précipité, le dicalcium phosphate, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges. Ces charges peuvent être traitées ou non en surface notamment pour les rendre lipophiles.
De préférence, les charges possèdent une granulométrie inférieure à 50 µm et représentent de 0,1 à 35%, de préférence de 0,5 à 25% et mieux de 1 à 15% du poids total de la première composition, si elles sont présentes.

***Agent de coloration***

La première composition du produit cosmétique de maquillage selon l'invention contient également un agent de coloration qui peut être choisi parmi les colorants hydrosolubles ou liposolubles, les pigments, les nacres et leurs mélanges.
Par « pigments », il faut comprendre des particules blanches ou colorées, minérales
ou organiques, insolubles dans la phase organique liquide, destinées à colorer et/ou opacifier la première composition.
Par « nacres », il faut comprendre des particules irisées, notamment produites par certains mollusques dans leur coquille ou bien synthétisées, insolubles dans le milieu de la première composition.
Par « colorants », il faut comprendre des composés généralement organiques solubles dans les corps gras comme les huiles ou dans une phase hydroalcoolique.
Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.
Les colorants hydrosolubles sont par exemple le jus de betterave, le bleu de méthylène, le caramel.
Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer (noir, jaune, brun ou rouge) ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium dont celles soumises à une certification par la Food and Drug Administration (FDA) (exemple D&C ou FD&C) et ceux exempts de la certification FDA comme les laques à base de carmin de cochenille.
Les nacres ou pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. On peut ainsi utiliser des pigments à propriétés goniochromatiques, notamment à cristaux liquides ou multicouches.
La seconde composition du produit selon l'invention peut également mais pas obligatoirement contenir un agent de coloration.
De manière générale, les agents de coloration représentent de 0,001 à 60 %, et mieux de 0,01 à 50 %, et mieux encore, de 0,1 à 40 % du poids total de chaque première et seconde composition.
L'agent de coloration ou la charge peuvent en outre être présents sous forme de « pâte particulaire ».

***Pâte particulaire***

Par « pâte particulaire », on entend au sens de l'invention une dispersion concentrée de particules enrobées ou non dans un milieu continu, stabilisée à l'aide d'un agent dispersant ou éventuellement sans agent dispersant. Ces

particules peuvent être choisies parmi les pigments, les nacres, les charges solides et leurs mélanges. Ces particules peuvent être de toute forme notamment de forme sphérique ou allongée comme des fibres. Elles sont insolubles dans le milieu.

L'agent dispersant sert à protéger les particules dispersées contre leur agglomération ou floculation. La concentration en dispersant généralement utilisée pour stabiliser une dispersion est de 0,3 à 5 mg/m$^2$, de préférence de 0,5 à 4 mg/m$^2$, de surface de particules. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise l'acide polyhydroxy-12-stéarique tel que celui vendu sous la référence Arlacel P100 par la société Uniqema, les esters de l'acide polyhydroxy-12-stéarique tel que le stéarate d'acide poly(12-hydroxystéarique) de poids moléculaire d'environ 750g/mole vendu sous le nom de Solsperse 21 000 par la société Avecia, les esters de l'acide polyhydroxy-12-stéarique avec des polyols tels que le glycérol, la diglycérine tels que le polygycéryl-2 dipolyhydroxystéarate (nom CTFA) vendu sous la référence Dehymuls PGPH par la société Henkel.

Comme autre dispersant utilisable dans la composition de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-3225 C.

L'acide polyhydroxy-12-stéarique et les esters de l'acide polyhydroxy-12-stéarique sont de préférence destinés à un milieu hydrocarboné ou fluoré, alors que les mélanges de diméthylsiloxane oxyéthylène/oxypropyléné sont de préférence destinés à un milieu siliconé.

La dispersion est une suspension de particules de taille généralement micronique (<10 μm) dans un milieu continu. La fraction volumique de particules dans une dispersion concentrée est de 20 % à 40 %, de préférence supérieure à 30 %, ce qui correspond à une teneur pondérale pouvant aller jusqu'à 70 % selon la densité des particules.

Les particules dispersées dans le milieu peuvent être constituées de particules minérales ou organiques ou de leurs mélanges tels que ceux décrits précédemment.

Le milieu continu de la pâte peut être quelconque et contenir tout solvant ou corps gras liquide et leurs mélanges. Avantageusement, le milieu liquide de la pâte particulaire est l'un des corps gras liquides ou huiles que l'on souhaite utiliser dans la première composition, faisant ainsi partie de la phase organique liquide de la première composition.

De façon avantageuse, la "pâte particulaire" est une "pâte pigmentaire" contenant une dispersion de particules colorées, enrobées ou non. Ces particules colorées sont des pigments, des nacres ou un mélange de pigments et/ou de nacres tels que ceux décrits plus haut.

De préférence, l'agent de coloration de la première composition est sous forme de dispersion ou pâte particulaire telle que décrite ci-dessus.

Avantageusement, la dispersion représente de 0,5 à 60 % en poids du poids total de chaque première ou seconde composition et mieux de 2 à 40 % et encore mieux de 2 à 30 %.

## Seconde composition

Le produit cosmétique de maquillage selon l'invention contient une seconde composition comprenant un second milieu physiologiquement acceptable.

Selon un mode préféré de réalisation de l'invention, le milieu physiologiquement acceptable de la seconde composition comprend une phase liquide non volatile à température ambiante et pression atmosphérique.

Par « phase liquide non volatile », on entend tout milieu susceptible de rester sur la peau ou les lèvres pendant plusieurs heures. Une phase liquide non volatile a en particulier une pression de vapeur à température ambiante et pression atmosphérique, non nulle inférieure à 0,02 mm de Hg (2,66 Pa) et mieux inférieure à $10^{-3}$ mm de Hg (0,13 Pa).

La phase liquide non volatile de la seconde composition peut être une phase hydrocarbonée liquide, une phase siliconée liquide et/ou une phase fluorée liquide à température ambiante.

De préférence, la phase liquide non volatile de la seconde composition sous forme hydrocarbonée est caractérisée par les paramètres de solubilité δD et δa selon l'espace de solubilité de Hansen satisfaisant aux conditions suivantes:

8 δD 22 (J/cm$^3$)$^{½}$, de préférence 12 δD 1 9 (J/cm$^3$)$^{½}$, et mieux 16 δD 19 ( J/cm$^3$)$^{½}$
et 7 δa 35 (J/cm$^3$)$^{½}$, de préférence 8 δa 2 0 (J/cm$^3$)$^{½}$, et mieux 8,5 δa 12 (J/cm$^3$)$^{½}$

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105

(1967) ;

- $\delta D$ caractérise les forces de dispersion de LONDON issues de la formation de dipôles induits lors des chocs moléculaires,

Et $\delta a=(\delta H^2+ \delta P^2)^{½}$ avec

- $\delta H$ qui caractérise les forces d'interactions spécifiques (type liaisons hydrogène, acide/base, donneur/accepteur, etc...) ;

- $\delta P$ qui caractérise les forces d'interactions de DEBYE entre dipôles permanents ainsi que les forces d'interactions de KEESOM entre dipôles induits et dipôles permanents.

Les paramètres $\delta D$ et $\delta a$ sont exprimés en $(J/cm^3)^{½}$.

La phase liquide non volatile peut être un mélange de différentes composés. Dans ce cas, les paramètres de solubilité du mélange sont déterminés à partir de ceux des composés pris séparément, selon les relations suivantes :

$$\delta_{Dmel} = \sum_i xi\, \delta_{Di} \quad ; \quad \delta_{pmel} = \sum_i xi\, \delta_{pi} \quad \text{et} \quad \delta_{hmel} = \sum_i xi\, \delta_{hi}$$

où xi représente la fraction volumique du composé i dans le mélange.

Il est à la portée de l'homme du métier de déterminer les quantités de chaque composé pour obtenir un mélange de corps gras satisfaisant aux relations ci-dessus.

Comme composés hydrocarbonées satisfaisant à ces paramètres de solubilité, on peut citer les composés suivants :

| | $\delta D$ | $\delta a$ |
|---|---|---|
| Diisostearyl Malate | 16,61 | 7,19 |
| Octyldodécanol | 16,36 | 7,70 |
| Propylene Glycol Monoisostearate | 16,36 | 8,74 |
| Polyglyceryl 2 Diisostearate | 16,79 | 9,07 |
| Huile de Ricin | 16,79 | 9,09 |
| Polyglyceryl 3 Diisostearate | 16,96 | 10,40 |
| Polyglyceryl 2 Isostearate | 17,03 | 13,25 |
| Butylene Glycol | 16,65 | 22,83 |
| Propylene Glycol | 15,95 | 25,02 |
| Glycérol | 17,81 | 31,73 |
| et leurs mélanges. | | |

Lorsque la phase liquide non volatile de la seconde composition contient une phase fluorée, elle comprend au moins un composé fluoré choisi parmi les composés fluorosiliconés, les polyéthers fluorés et/ou les alcanes fluorés.

De préférence, la phase liquide non volatile de la seconde composition comprend au moins un composé fluorosiliconé de formule (I):

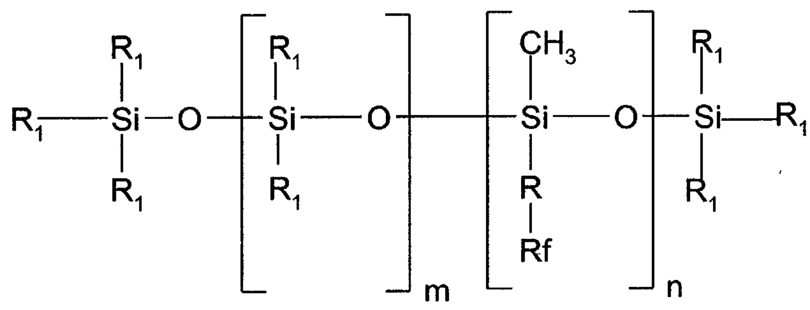

(I)

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- $R_1$ représentent, indépendamment l'un de l'autre, un radical alkyle en $C_1$-$C_{20}$, un radical hydroxyle, un radical phényle,
- m est choisi de 0 à 150, de préférence de 20 à 100, et
- n est choisi de 1 à 300, de préférence de 1 à 100.

On peut notamment citer comme composés fluorosiliconés de formule (I) ceux qui sont commercialisés par la société Shin-Etsu sous les dénominations 'X22-819', 'X22-820', 'X22-821' et 'X22-822' ou encore 'FL-100'.
Comme autre composé fluoré pouvant entrer dans la composition de la phase fluorée de la seconde composition, on peut notamment citer les polyéthers fluorés de formule (II) suivante :

$$R_6 - (CF_2\text{-}CFR_3\text{-}CF_2O)p - (CFR_4\text{-}CF_2\text{-}O)q - (CFR_5\text{-}O)r - R_7 \qquad \text{(II)}$$

dans laquelle :

- $R_3$ à $R_6$ représentent, de manière indépendante l'un de l'autre, un radical monovalent choisi parmi -F, $-(CF_2)n-CF_3$, et $-O-(CF_2)n-CF_3$,
- $R_7$ représente un radical monovalent choisi parmi -F et $-(CF_2)n-CF_3$,
- avec n allant de 0 à 4 inclus,
- p allant de 0 à 600, q allant de 0 à 860, r allant de 0 à 1500, et p, q et r étant des entiers choisis de manière à ce que la masse moléculaire en poids du composé soit va de 500 à 100000, de préférence entre 500 à 10000.

De tels composés sont notamment décrits dans le brevet EP-A-196904.
Parmi les produits commerciaux utilisables dans la présente invention comme composé fluoré, on peut citer les Fomblins de la société Montefluos, et les Demnum S de la société Daikin Industries.
On peut également citer, comme composés fluorés susceptibles d'être utilisés dans le cadre de la présente invention, les alcanes fluorés, tels que les perfluoroalcanes et les fluoroalcanes en $C_2$-$C_{50}$, et notamment ceux en $C_5$-$C_{30}$, tels que la perfluorodécaline, le perfluoroadamantane et le bromoperfluorooctyle et leurs mélanges.
Lorsque la phase liquide non volatile de la seconde composition contient une phase siliconée, elle contient avantageusement au moins une huile siliconée et de préférence une huile siliconée phénylée.
Les huiles siliconées phénylées utilisables selon la présente invention présentent une viscosité mesurée à 25°C et pression atmosphérique allant de 5 à 100 000 cSt, et de préférence de 5 à 10 000 cSt.
L'huile siliconée peut être par exemple une phényl trimethicone, une phényl dimethicone, une phényl triméthylsiloxy diphénylsiloxane, une diphényl diméthicone, une diphényl méthyldiphényl trisiloxane, ou un mélange de différentes huiles siliconées phénylées, et en particulier peut répondre à la formule (A) suivante :

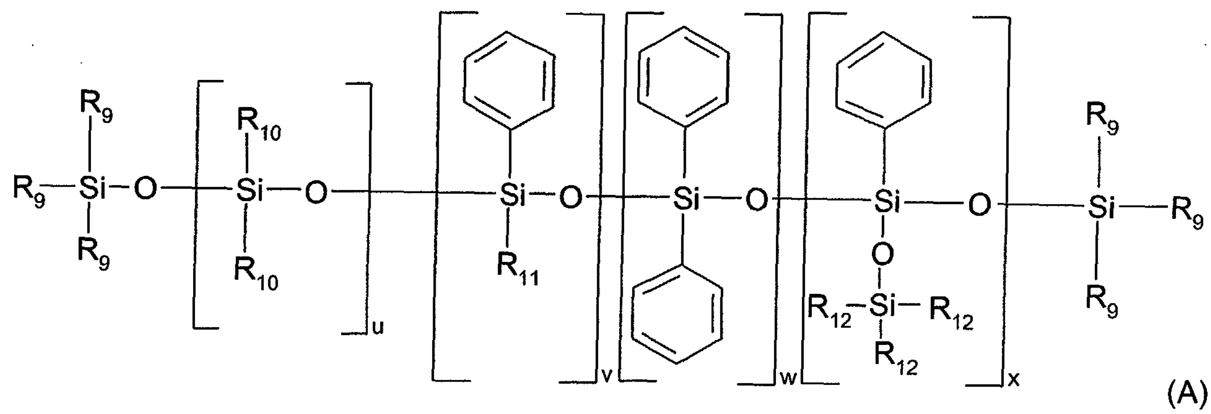

(A)

dans laquelle

- $R_9$ et $R_{12}$ sont chacun indépendamment un radical alkyle en $C_1$-$C_{30}$, un radical aryle ou un radical aralkyle,
- $R_{10}$ et $R_{11}$ sont chacun indépendamment un radical alkyle en $C_1$-$C_{30}$ ou un radical aralkyle,
- u, v, w et x sont chacun indépendamment des nombres entiers allant de 0 à 900, sous réserve que la somme v+w+x soit différente de 0 et que la somme u+v+w+x va de 1 à 900, en particulier, u+v+w+x va de 1 à 800.

Avantageusement, $R_9$ est un radical alkyle en $C_1$-$C_{20}$, un radical phényle ou un radical aralkyle du type R'-$C_6H_5$, R' étant un alkyle en $C_1$-$C_5$, $R_{10}$ et $R_{11}$ sont chacun indépendamment un radical alkyle en $C_1$-$C_{20}$ ou un radical aralkyle du type R'-$C_6H_5$, R' étant un alkyle en $C_1$-$C_5$, et $R_{12}$ est un radical alkyle en $C_1$-$C_{20}$.

De préférence, $R_9$ est un radical méthyle, éthyle, propyle, isopropyle, décyle, dodécyle ou octadécyle, ou encore un radical phényle, tolyle, benzyle ou phénéthyle, $R_{10}$ et $R_{11}$ sont chacun indépendamment un radical méthyle, éthyle, propyle, isopropyle, décyle, dodécyle ou octadécyle, ou encore un radical tolyle, benzyle ou phénéthyle, et $R_{12}$ est un radical méthyle, éthyle, propyle, isopropyle, décyle, dodécyle ou octadécyle.

Suivant un mode de réalisation préféré de l'invention, la phase liquide non volatile de la seconde composition contient une phase siliconée comprenant une huile siliconée phénylée ayant une viscosité inférieure à 500 cSt à 25°C, appelée « huile siliconée phénylée de basse viscosité », et une huile siliconée phénylée ayant une viscosité au moins égale à 500 cSt à 25°C, appelée « huile siliconée phénylée de haute viscosité ». Avantageusement, l'huile siliconée phénylée de basse viscosité présente une viscosité à 25°C allant par exemple de 5 à 499 cSt, de préférence de 5 à 300 cSt, et mieux de 5 à 100 cSt, et l'huile siliconée phénylée de haute viscosité présente une viscosité à 25 °C allant par exemple de 500 à 10 000 cSt, de préférence de 600 à 5000 cSt, et mieux de 600 à 3000 cSt.

L'utilisation d'huiles siliconées phénylées de basse et haute viscosités telles que définies ci-dessus permet d'obtenir, après dépôt sur la peau, les lèvres et/ou les phanères, un film de composition particulièrement brillant, homogène et de bonne tenue.

De préférence, ces huiles siliconées phénylées de basse et haute viscosités satisfont à la formule (A). De préférence, la première huile siliconée phénylée de basse viscosité satisfait à la formule (A) avec la somme u+v+w+x allant de 1 à 150, et mieux de 1 à 100, voire de 1 à 50 et la seconde huile siliconée phénylée de haute viscosité satisfait à la formule (A) avec la somme u+v+w+x allant de 151 à 900, mieux de 160 à 800, voire de 160 à 500.

En particulier, l'huile siliconée phénylée de basse viscosité satisfait à la formule (III) suivante :

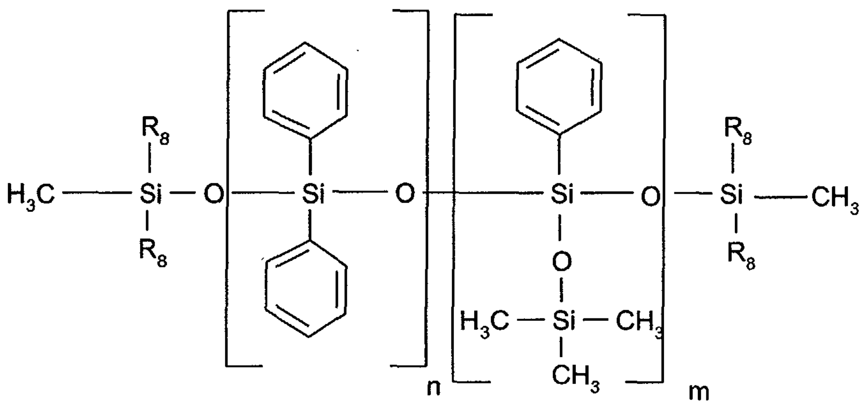

(III)

dans laquelle

- . $R_8$ est un radical alkyle en $C_1$-$C_{30}$, un radical aryle, ou un radical aralkyle,
- . n est un nombre entier allant de 0 à 100, et mieux, inférieur à 100,
- . m est un nombre entier allant de 0 à 100, sous réserve que la somme m+n va de 1 à 100 , et mieux, est inférieure à 100.

Avantageusement, $R_8$ est un radical alkyle en $C_1$-$C_{20}$, un radical phényle ou un radical aralkyle du type R'-$C_6H_5$, R' étant un alkyle en $C_1$-$C_5$.

De préférence, $R_8$ est un radical méthyle, éthyle, propyle, isopropyle, décyle, dodécyle ou octadécyle, ou encore un radical phényle, tolyle, benzyle ou phénéthyle.
Avantageusement, $R_8$ est un radical méthyle.

Parmi les huiles siliconées phénylées de basse viscosité utilisables dans l'invention, on peut citer les huiles DC556 (22,5 cSt), SF558 (10-20 cSt) de Dow Corning, l'huile Abil AV8853 (4-6 cSt) de Goldschmidt, l'huile Silbione 70 633 V 30 (28 cSt) de Rhône Poulenc, les huiles 15 M 40 (50 à 100 cSt), 15 M 50 (20 à 25 cSt) de PCR, les huiles SF 1550 (25 cst), PK 20 (20 cSt) de Bayer, l'huile Belsil PDM 200 (200cst) de Wacker, les huiles KF 53 (175 cSt), KF 54 (400 cSt) et KF 56 (14 cSt) de Shin-Etsu.
Parmi les huiles siliconées phénylées de haute viscosité utilisables dans l'invention, on peut citer les huiles 15 M 30 de PCR (500 cSt) ou la Belsil PDM 1000 (1000 cSt) de Wacker.
Les valeurs entre parenthèses représentent les viscosités à 25°C.

Le rapport en poids entre l'huile siliconée phénylée de basse viscosité et l'huile siliconée phénylée de haute viscosité peut aller par exemple de 70/30 à 30/70, mieux de 60/40 à 40/60 et encore mieux de 55/45 à 45/55.

De préférence, la phase liquide non volatile de la seconde composition est une phase siliconée liquide à température ambiante.

La phase liquide non volatile de la seconde composition représente de 1 à 100%, de préférence de 5 à 95%, mieux de 20 à 80% et mieux encore, de 40 à 80 % du poids total de la seconde composition.

Le milieu physiologiquement acceptable de la seconde composition peut contenir une phase liquide volatile dont la vitesse d'évaporation est différente de la vitesse d'évaporation de la phase volatile de la première composition, et notamment la vitesse d'évaporation de la phase volatile de la seconde composition est inférieure à la vitesse d'évaporation de la phase volatile de la première composition.

***Additifs***

Les première et/ou seconde compositions du produit de maquillage selon l'invention peuvent également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines( C, A, E, F, B, ou PP), acides gras essentiels, huiles essentielles, céramides, sphingolipides, filtres solaires liposolubles ou sous forme de nanoparticules, les actifs spécifiques de traitement de la peau (agents de protection, anti-bactériens, anti-rides...).

Ces actifs sont utilisés en quantité habituelle pour l'homme de l'art et notamment à des concentrations de 0 à 20 % et notamment de 0,001 à 15 % du poids total de la première et/ou seconde composition.

Chaque composition du produit selon l'invention peut, de plus, comprendre, selon le type d'application envisagée, les constituants classiquement utilisés dans les domaines considérés, qui sont présents en une quantité appropriée à la forme galénique souhaitée.

De manière générale, les milieux physiologiquement acceptables de chacune des première et seconde compositions du produit selon l'invention peuvent comprendre, outre la phase liquide organique, la dispersion de polymère et l'agent de coloration pour la première composition et la phase liquide non volatile pour la seconde composition, des corps gras additionnels qui peuvent être choisis parmi les cires, les huiles, les gommes et/ou les corps gras pâteux, hydrocarbonés, siliconés et/ou fluorés, d'origine végétale, animale, minérale ou de synthèse et leurs mélanges.

De préférence, le milieu physiologiquement acceptable de la première composition contient une gomme, de préférence une gomme de silicone ayant une viscosité à température ambiante allant de 50 000 à $10^7$ cSt, de préférence de 100 000 à $10^6$ cSt.

De préférence, le milieu physiologiquement acceptable de la première et/ou de la seconde composition contient un corps gras pâteux et/ou une cire choisie parmi les cires précédemment citées.

Chaque composition du produit selon l'invention peuvent contenir, en outre, tout additif usuellement utilisé dans de telles compositions, comme des épaississants d'huile ou de phase aqueuse (gélifiant acrylique), des antioxydants, des parfums, des conservateurs (pentylène glycol), des tensioactifs, des polymères liposolubles (copolymère de polyvinylpyrrolidone/eicosène par exemple).

Lorsque le milieu physiologiquement acceptable de la première et/ou de la seconde composition contient une phase organique liquide, ce milieu peut notamment contenir de l'eau dispersée ou émulsionnée dans ladite phase organique liquide.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, sous forme de pâte souple dans une bouillotte, ou sous la forme de coupelle utilisable par contact direct ou à l'éponge. En particulier, elles constituent, ensemble ou séparément, un fond de teint coulé, fard à joues ou à paupières coulé notamment coloré, rouge à lèvres, brillant pour les lèvres, produit anti-cernes. Elles peuvent aussi se présenter chacune sous forme d'une pâte souple ou encore de gel, de crème plus ou moins fluide. Elles peuvent alors constituer des fonds de teint ou des rouges à lèvres fluides ou pâteux, des brillants à lèvres, des produits solaires ou de coloration de la peau, des eyeliners, des produits de maquillage du corps ou encore présenter des propriétés de soin et se présenter alors sous forme de base ou de baume de soin des lèvres.

Chaque composition du produit selon l'invention peuvent se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Avantageusement, la première ou la seconde composition, ou les deux, sont à phase grasse continue et de préférence sous forme anhydre et peuvent contenir moins de 5% d'eau, et encore mieux moins de 1% d'eau par rapport au poids total de la première ou seconde composition. En particulier, le produit de maquillage bicouche entier est sous forme anhydre.

Chaque première et seconde composition peut avoir l'aspect d'une lotion, d'une crème, d'une pommade, d'une pâte souple, d'un onguent, d'un solide coulé ou moulé et notamment en stick ou en coupelle, ou encore de solide compacté.

De préférence, chaque composition se présente sous la forme d'un stick plus ou moins rigide.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

De préférence, la composition qui est appliquée en première couche est sous forme solide, ce qui permet une application plus pratique, une meilleure stabilité dans le temps et en température de la composition et permet un tracé précis du maquillage, ce qui est hautement souhaitable dans le cas d'un rouge à lèvres ou d'un eyeliner.

Le produit selon l'invention peut être avantageusement utilisé pour le maquillage de la peau et/ou des lèvres et/ou des phanères selon la nature des ingrédients utilisés. En particulier, le produit de l'invention peut être se présenter sous forme, de fond de teint solide, de bâton ou pâte de rouge à lèvres, de produit anti-cernes, ou contours des yeux, d'eye liner, de mascara, d'ombre à paupières, de produit de maquillage du corps ou encore un produit de coloration de la peau.

Le produit est en particulier un rouge à lèvres.

De préférence, la première et/ou la seconde composition est sous forme solide.

Avantageusement, la couche du dessus présente des propriétés de soin.

L'invention a encore pour objet un produit à lèvres, un fond de teint, un tatouage, un fard à joues ou à paupières contenant une première et une seconde compositions telles que décrites précédemment.

Les compositions de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-0667 146.

Les exemples ci-dessous ont pour but d'illustrer de manière non limitative la présente invention. Les quantités sont données en pourcentages massiques .

**Exemple 1 : Dispersion de polymère**

On prépare une dispersion de copolymère non réticulé d'acrylate de méthyle et d'acide acrylique dans un rapport 95/5 dans de l'isododécane, selon la méthode de l'exemple 1 du document EP-A-749 746, en remplaçant l'heptane par de l'isododécane. On obtient ainsi une dispersion de particules de poly(acrylate de méthyle/acide acrylique) stabilisées en surface dans de l'isododécane par un copolymère dibloc séquencé polystyrène/copoly(éthylène-propylène) vendu sous le nom de KRATON G1701 (Shell), ayant un taux de matière sèche de 24,6% en poids et une taille moyenne des particules de 180 nm (polydispersité : 0,05%) et une Tg de 20°C. Ce copolymère est filmifiable.

**Exemple 2 : Pâte pigmentaire**

La pâte pigmentaire utilisée est un mélange de 3 pâtes pigmentaires contenant chacune un pigment différent :

| *Pâte n°1 :* | |
|---|---|
| DCRed N°7 | 30% |
| Stéarate d'acide poly(12-hydroxystéarique )(Solsperse 21000) | 2% |
| Polyisobutène Hydrogéné (Parléam) | 68% |

| *Pâte n°2 :* | |
|---|---|
| Yellow N°6 AL Lake | 50% |
| Stéarate d'acide poly(12-hydroxystéarique) (Solsperse 2100) | 2% |
| polyisobutène hydrogéné (Parléam) | 48% |

| *Pâte n°3 :* | |
|---|---|
| Dioxyde de titane | 70% |
| Stéarate d'acide poly(12-hydroxystéarique (Solsperse 21000) | 1% |
| polyisobutène hydrogéné (Parléam) | 29% |

On réalise une pâte pigmentaire contenant un mélange de 10% de la pâte n°1, 2% de la pâte n°2 et 2,14%de la pâte n°3.

**Exemple 3 : Produit pour le maquillage des lèvres**

Première composition

*Phase A*

- dispersion de particules de polymère de l'exemple 1 71%
- copolymère éthylène /octène (76/24) vendu sous la référence ENGAGE 8400 par la société Dupont de Nemours 3,50%

*Phase B*

- pâte pigmentaire de l'exemple 2 14,14%

*Phase C*

- polytétrafluoroéthylène 10 %

*Phase D*

- cyclopentasiloxane 1,36%

Mode opératoire

La phase A est obtenue en dissolvant le gélifiant (copolymère éthylène/octène) à 110°C dans la dispersion de particules de polymère pendant environ une heure à l'aide d'un agitateur Raynerie.
Après homogénéisation, on laisse la température revenir vers 30°C puis on ajoute successivement les phases B, C et D sous agitation à l'aide d'un agitateur Raynerie. La première composition est ensuite conditionnée dans une bouillotte à température ambiante. Elle est sous forme de pâte souple .

Seconde composition

- huile de ricin 81,00%
- PEG 45 dodecylglycol copolymère 10,00%
- stéarate d'octacosanyle (cire) 9,00%

Mode opératoire

Les constituants sont pesés ensemble et chauffés à 100°C jusqu'à fusion complète de la cire.
Après homogénéisation on peut couler la composition dans un moule adéquat pour obtenir un stick sous la forme de « stylo ».
On applique sur les lèvres une première couche de la première composition puis on laisse sécher 3 minutes.
On applique ensuite sur la première couche une seconde couche de la seconde composition .
On obtient ainsi un maquillage bicouche brillant, confortable, de bonne tenue, qui ne migre pas et ne transfère pas.
Ces propriétés ont été vérifiées et confirmées par des personnes qualifiées.

**Exemple 4 : Produit pour le maquillage des lèvres**

Première composition

*Phase A*

- dispersion de particules de polymère de l'exemple 1 71%
- PVP/Eicosene copolymer 3,5%

*Phase B*

- pâte pigmentaire de l'exemple 2 14,14%

*Phase C*

- polytétrafluoroéthylène 10%

*Phase D*

- cyclopentasiloxane 1,36%

On utilise le même mode opératoire que celui de la première composition de l'exemple 3.

Seconde composition

- Phenyltrimethicone (de viscosité égale à 20cSt) vendue sous la référence DC 556 par la société Dow Corning 46%
- Phenyltrimethicone (de viscosité égale à 1000 cSt) vendue sous la référence BELSIL PDM 1000 par la société WACKER 46%
- cire de polyéthylène (Mw*=500) 8%

Mw représente la masse moléculaire moyenne en poids.

Mode opératoire

Les constituants sont pesés ensemble et chauffés à 100°C jusqu'à fusion complète de la cire. Après homogénéisation, on peut couler la composition dans un moule adéquat pour obtenir un stick sous la forme de « stylo ».

On applique ensuite sur les lèvres une première couche de la première composition à l'aide d'un feutre que l'on laisse sécher environ 3 minutes. On applique ensuite sur cette première couche une seconde couche de la seconde composition.

On obtient un maquillage des lèvres bicouche brillant, confortable, non desséchant, de bonne tenue, qui ne migre pas et ne transfère pas sur une tasse ou un tissu après séchage de 2 minutes environ. Ces propriétés ont été vérifiées et confirmées par des personnes qualifiées.

**Exemple 5 : Produit pour le maquillage des lèvres**

Première composition

*Phase A*

- dispersion de particules de polymère de l'exemple 1 75,5%
- copolymère éthylène /octène (76/24) vendu sous la référence ENGAGE 8400 par la société Dupont de Nemours 3,5%

*Phase B*

- pâte pigmentaire de l'exemple 2 14,14%

*Phase C*

- polytétrafluoroéthylène 5%

*Phase D*

- cyclopentasiloxane 1,36%

On utilise le même mode opératoire de fabrication et d'application sur les lèvres que celui de la première composition de l'exemple 3.

Seconde composition

- Phenyltrimethicone (de viscosité égale à 20cSt) vendue sous la référence DC 556 par la société Dow Corning 42%
- Phenyltrimethicone (de viscosité égale à 1000 cSt) vendue sous la référence BELSIL PDM 1000 par la société WACKER 42%
- silicone fluorée vendue sous la référence X 22819 par la société Shin-Etsu 8%
- cire de polyéthylène (Mw=500) 8%

Mode opératoire

Les constituants de la seconde composition sont pesés ensemble et chauffés à 100°C jusqu'à fusion complète de la cire.
Après homogénéisation, on peut couler la composition dans un moule adéquat pour obtenir un stick sous la forme de « stylo ».
On applique sur les lèvres une première couche de la première composition à l'aide d'un feutre que l'on laisse sécher environ 3 minutes. On applique ensuite sur cette première couche une seconde couche de la seconde composition.
On obtient un maquillage des lèvres bicouche brillant, confortable (non tiraillant), de bonne tenue, qui ne migre pas et ne transfère pas et se démaquille aisément. Ces propriétés ont été vérifiées et confirmées par des personnes qualifiées.

**Exemple 6 : Produit pour le maquillage des lèvres**

Première composition

*Phase A*

- dispersion de particules de polymère de l'exemple 1 71%
- cire de polyéthylène vendue sous la référence AC 617 par la société Honeywell 3,5%

*Phase B*

- pâte pigmentaire de l'exemple 2 14,14%

*Phase C*

- polytétrafluoroéthylène 10%

*Phase D*

- cyclopentasiloxane 1,36%

On utilise le même mode opératoire de fabrication et d'utilisation que la première composition de l'exemple 3.

On applique en surcouche la seconde composition de l'exemple 4. L'utilisation du produit est conforme à l'exemple 4, il en est de même pour ses propriétés cosmétiques de maquillage.

**Exemple 7 : Produit pour le maquillage des lèvres**

Première composition

*Phase A*

- dispersion de particules de polymère de l'exemple 1 75%
- Hectorite modifiée par du chlorure de di-stearyldimethyl ammonium 3,38% vendu sous la référence BENTONE 38 VCG par la société Elementis
- propylène carbonate 1,12%

*Phase B*

- pâte pigmentaire de l'exemple 2 14,14%

*Phase C*

- polytétrafluoroéthylène 5%

*Phase D*

- cyclopentasiloxane 1,36%

Mode opératoire

La phase A est obtenue en ajoutant lentement l'Hectorite modifiée sous agitation à l'aide d'un agitateur Raynerie à température ambiante (25°C).
On ajoute ensuite le propylène carbonate en maintenant l'agitation.
Après homogénéisation, on ajoute successivement les phases B, C et D à température ambiante toujours sous agitation à l'aide d'un agitateur Raynerie.
La composition est ensuite conditionnée dans une bouillotte à température ambiante. Elle est sous forme de pâte souple et est appliquée avec un feutre.

On applique cette première composition sur les lèvres puis on la laisse sécher 3 minutes.

On applique en surcouche la seconde composition de l'exemple 4, selon la même mise en oeuvre que dans

cet exemple. Le maquillage bicouche obtenu présente les mêmes propriétés de confort, de tenue, de non transfert et de non migration que celui du produit de l'exemple 3.

**Exemple 8 : Produit pour le maquillage des lèvres**

Première composition

*Phase A*

- dispersion de particules de polymère de l'exemple 1 69,16%

*Phase B*

- pâte pigmentaire de l'exemple 2 14,14%

*Phase C*

- cire de polyéthylène (Mw=500) 12%
- ozokerite 3,20%
- alcool gras linéaire vendu sous la référence PERFORMACOL 550 par la société New Phase Technologies 1,50%

Mode opératoire

L'ensemble des constituants de la première composition sont pesés et mélangés à 100-105°C à l'aide d'un agitateur Raynerie.
Après homogénéisation, la composition est coulée à 100°C dans un moule et conditionnée sous forme de « stylo ».
On applique ensuite cette première composition sur les lèvres puis on laisse sécher 3 minutes.
On applique en surcouche la seconde composition de l'exemple 4 selon la même mise en oeuvre. Les propriétés cosmétiques de maquillage du produit sont conformes à celles du produit de l'exemple 4.

**Exemple 9 : Fards à paupières**

Première composition

- dispersion de particules de polymère de l'exemple 1 75,6%
- huile de parléam 6,4%
- poudre de Nylon 8%
- bleu outremer 10%

Les différents ingrédients sont mélangés à température ambiante à l'aide d'un agitateur type Raynerie.
On applique sur chaque paupière une première couche de cette première composition. On laisse sécher environ 3 minutes puis on applique en surcouche sur les paupières la seconde composition de l'exemple 3.
On obtient un maquillage bicouche brillant, confortable, de bonne tenue, qui ne migre pas dans les plis et ridules des paupières et ne transfère pas sur un tissu mis au contact des yeux.
Les produits de maquillage des exemples ci-dessus se démaquillent facilement avec un démaquillant waterproof classique tel que le BIFACIL commercialisé par la Société Lancôme.

## Revendications

1. Produit cosmétique de maquillage contenant une première et une seconde compositions, la première composition comprenant, dans un milieu physiologiquement acceptable, des particules de polymère dispersées et stabilisées en surface par un stabilisant dans une phase organique liquide et un agent de coloration, et la seconde composition comprenant un second milieu physiologiquement acceptable.

2. Produit selon la revendication précédente, **caractérisé en ce que** les particules de polymère ont une taille moyenne comprise entre 5 et 800 nm.

3. Produit selon l'une des revendications précédentes, **caractérisé en ce que** les particules de polymère sont insolubles dans les alcools hydrosolubles.

4. Produit selon l'une des revendications précédentes, **caractérisé en ce que** les particules de polymère sont choisies parmi les polyuréthanes, polyuréthanes-acryliques, polyurées, polyurée/polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

5. Produit selon l'une des revendications précédentes, **caractérisé en ce que** le polymère est filmifiable.

6. Produit selon l'une des revendications précédentes, **caractérisé en ce que** le polymère représente, en matière sèche, de 2 à 40% du poids total de la première composition, de préférence de 5 à 30% et mieux encore de 8 à 20%.

7. Produit selon l'une des revendications précédentes, **caractérisé en ce que** le stabilisant est choisi parmi les polymères séquencés, les polymères greffés, les polymères statistiques, et leurs mélanges.

8. Produit selon l'une des revendications précédentes, **caractérisé en ce que** le stabilisant est choisi parmi les polymères siliconés greffés avec une chaîne hydrocarbonée; les polymères hydrocarbonés greffés avec une chaîne siliconée; les copolymères greffés ayant un squelette insoluble de type polyacrylique avec des greffons solubles de type acide poly-12(hydroxystéarique); les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins un bloc d'un polyéther ; les copolymères d'acrylates ou de méthacrylates d'alkyle en $C_1$-$C_4$, ou d'acrylates ou de méthacrylates d'alkyle en $C_8$-$C_{30}$; les copolymères blocs greffés ou séquencés comprenant au moins un bloc résultant de la polymérisation de monomères éthyléniques comportant éventuellement des liaisons conjuguées et au moins un bloc d'un polymère vinylique ; les copolymères blocs greffés ou séquencés comprenant au moins un bloc résultant de la polymérisation de monomères éthyléniques comportant éventuellement des liaisons conjuguées et au moins un bloc d'un polymère acrylique ; les copolymères blocs greffés ou séquencés comprenant au moins un bloc résultant de la polymérisation de diène et au moins un bloc d'un polyéther, et leurs mélanges.

9. Produit selon l'une des revendications précédentes, **caractérisé en ce que** le stabilisant est un polymère bloc greffé ou séquencé, comprenant au moins un bloc résultant de la polymérisation de diène et au moins un bloc d'un polymère vinylique.

10. Produit selon l'une des revendications précédentes, **caractérisé en ce que** le stabilisant est un polymère dibloc.

11. Produit selon l'une des revendications précédentes, **caractérisé en ce que** la phase organique liquide de la première composition contient au moins une huile organique volatile à température ambiante et pression atmosphérique.

12. Produit selon l'une des revendications précédentes, **caractérisé en ce que** l'huile organique volatile représente de 20 à 90% et de préférence de 30 à 80 %, et mieux de 40 à 70% du poids total de la première composition.

13. Produit selon l'une des revendications précédentes, **caractérisé en ce que** l'agent de coloration est choisi parmi les colorants liposolubles, les colorants hydrosolubles, les pigments, les nacres et leurs mélanges.

14. Produit selon l'une des revendications précédentes, **caractérisée en ce que** l'agent de coloration est présent à

raison de 0,001 à 60 % du poids total de la première composition, de préférence de 0,01 à 50 % et mieux de 0,1 à 40%.

**15.** Produit selon l'une des revendications précédentes, **caractérisé en ce que** l'agent de coloration est sous forme de dispersion.

**16.** Produit selon l'une des revendications précédentes, **caractérisé en ce que** la première composition comprend en outre un agent rhéologique structurant et/ou gélifiant de son milieu physiologiquement acceptable, ledit agent étant choisi parmi les gélifiants lipophiles, les cires, les charges et leurs mélanges.

**17.** Produit selon la revendication 16, **caractérisé en ce que** l'agent rhéologique représente de 0,1 à 50 % du poids total de la première composition et mieux de 1 à 25 %.

**18.** Produit selon la revendication 16 ou 17, **caractérisé en ce que** l'agent rhéologique comprend un gélifiant lipophile choisi parmi les homopolymères ou copolymères de l'éthylène dont la masse moléculaire en poids est comprise entre 300 et 500 000, et mieux entre 500 et 100 000 et leurs mélanges.

**19.** Produit selon l'une des revendications 16 à 18, **caractérisé en ce que** l'agent rhéologique est choisi parmi les copolymères d'oléfines à cristallisation contrôlée et leurs mélanges.

**20.** Produit selon l'une des revendications 16 à 19, **caractérisé en ce que** l'agent rhéologique est un copolymère éthylène/octène.

**21.** Produit selon l'une des revendications 16 à 20, **caractérisé en ce que** l'agent rhéologique représente de 0,5 à 20%, et mieux de 1 à 10% du poids total de la première composition.

**22.** Produit selon l'une des revendications 16 à 21, **caractérisé en ce que** l'agent rhéologique comprend une cire.

**23.** Produit selon la revendication 22, **caractérisé en ce que** la cire est présente à raison de 0,1 à 50% en poids dans la première composition et mieux de 3 à 25%.

**24.** Produit selon l'une des revendications 16 à 23, **caractérisé en ce que** l'agent rhéologique comprend une charge.

**25.** Produit selon la revendication 24, **caractérisé en ce que** la charge possède une granulométrie inférieure à 50 μm.

**26.** Produit selon la revendication 24 ou 25, **caractérisé en ce que** la charge est choisie parmi le talc, le mica, la silice, le kaolin, les poudres de polyamide, de poly-β-alanine et de polyéthylène, les poudres de polymères de tétrafluoroéthylène, la lauroyl-lysine, l'amidon, le nitrure de bore, les microsphères polymériques creuses, les particules de polymère acrylique, les microbilles de résine de silicone, le carbonate de calcium précipité, le dicalcium phosphate, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone et leurs mélanges.

**27.** Produit selon l'une des revendications 24 à 26, **caractérisé en ce que** la charge représente de 0,1 à 35%, de préférence de 0,5 à 25% et mieux de 1 à 15% du poids de la première composition.

**28.** Produit selon l'une des revendications précédentes, **caractérisé en ce que** le milieu physiologiquement acceptable de la seconde composition comprend une phase liquide non volatile à température ambiante et pression atmosphérique.

**29.** Produit selon la revendication 28, **caractérisé en ce que** la phase liquide non volatile de la seconde composition contient une phase hydrocarbonée liquide, une phase fluorée liquide et/ou une phase siliconée liquide.

**30.** Produit selon la revendication 29, **caractérisé en ce que** la phase liquide non volatile de la seconde composition contient une phase hydrocarbonée présentant les paramètres de solubilité δD et δa selon l'espace de solubilité de Hansen satisfaisant aux conditions suivantes:

8 δD 22 $(J/cm^3)^{1/2}$, de préférence 12 δD 1 9 $(J/cm^3)^{1/2}$, et mieux 16 δD 19 $( J/cm^3)^{1/2}$

et 7 δa 35 $(J/cm^3)^{½}$, de préférence 8 δa 2 0 $(J/cm^3)^{½}$, et mieux 8,5 δa 12 $(J/cm^3)^{½}$

**31.** Produit selon la revendication 29 ou 30, **caractérisé en ce que** la phase hydrocarbonée est composé d'au moins un composé hydrocarboné choisie parmi les composés suivants : diisostéaryl malate, octyldodécanol, propylene glycol monoisostéarate, polyglycéryl 2 diisostearate, huile de ricin, polyglyceryl 3 diisostéarate, polyglycéryl 2 isostearate, butylene glycol, propylene glycol, glycérol et leurs mélanges.

**32.** Produit selon la revendication 29, **caractérisé en ce que** la phase liquide non volatile de la seconde composition contient une phase fluorée comprenant au moins un compose fluoré choisi parmi les composés fluorosiliconés, les polyéthers fluorés et/ou les alcanes fluorés.

**33.** Produit selon la revendication 32, **caractérisé en ce que** les composés fluorosiliconés sont choisis parmi les composés de formule (I) suivante :

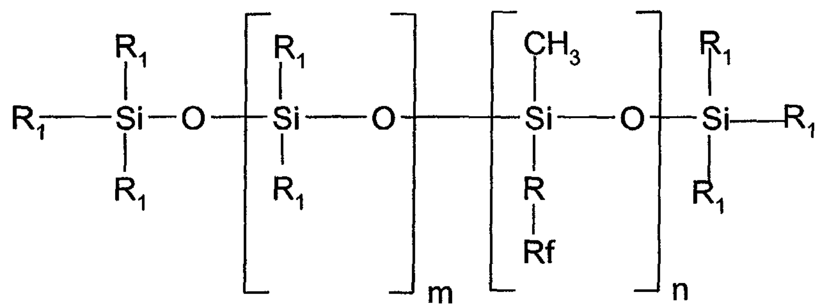
(I)

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- $R_1$ représentent, indépendamment l'un de l'autre, un radical alkyle en $C_1$-$C_{20}$, un radical hydroxyle, un radical phényle,
- m est choisi de 0 à 150, de préférence de 20 à 100, et
- n est choisi de 1 à 300, de préférence de 1 à 100.

**34.** Produit selon la revendication 32, **caractérisé en ce que** les polyéthers fluorés sont choisis parmi les composé de formule (II) suivante :

$$R_6 - (CF_2\text{-}CFR_3\text{-}CF_2O)p - (CFR_4\text{-}CF_2\text{-}O)q - (CFR_5\text{-}O)r - R_7 \quad (II)$$

dans laquelle :

- $R_3$ à $R_6$ représentent, de manière indépendante l'un de l'autre, un radical monovalent choisi parmi -F, $-(CF_2)n-CF_3$, et $-O-(CF_2)n-CF_3$,
- $R_7$ représente un radical monovalent choisi parmi -F et $-(CF_2)n-CF_3$,
- avec n allant de 0 à 4 inclus,
- p allant de 0 à 600, q allant de 0 à 860, r allant de 0 à 1500, et p, q et r étant des entiers choisis de manière à ce que la masse moléculaire en poids du composé va de 500 à 100000, de préférence de 500 à 10000.

**35.** Produit selon la revendication 32, **caractérisé en ce que** les alcanes fluorés sont choisi parmi les perfluoroalcanes et les fluoroalcanes en C2-C50, de préférence en C5-C30, tels que la perfluorodécaline, le perfluoroadamantane et le bromoperfluorooctyle et leurs mélanges.

**36.** Produit selon l'une quelconque des revendications 28 à 35, **caractérisé en ce que** la phase liquide non volatile

de la seconde composition contient une phase siliconée composée d'au moins une huile siliconée.

**37.** Produit selon la revendication 36, **caractérisé en ce que** l'huile siliconée est une huile siliconée phénylée présentant une viscosité mesurée à 25°C allant de 5 à 100 000 cSt et de préférence de 5 à 10 000 cSt.

**38.** Produit selon la revendication 36 ou 37, **caractérisé en ce que** l'huile siliconée est choisie parmi les huiles siliconées phénylées de formule (A) suivante :

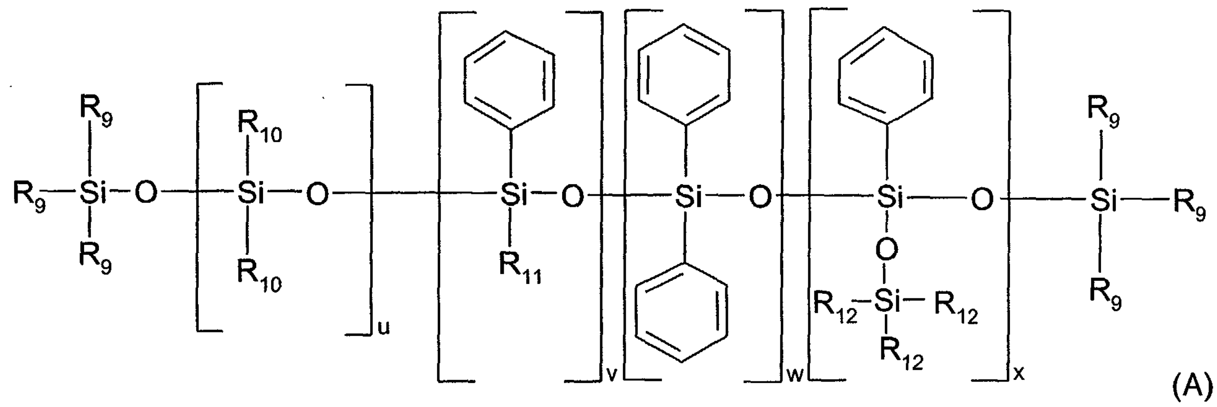

(A)

dans laquelle

- $R_9$ et $R_{12}$ sont chacun indépendamment un radical alkyle en $C_1$-$C_{30}$, un radical aryle ou un radical aralkyle,
- $R_{10}$ et $R_{11}$ sont chacun indépendamment un radical alkyle en $C_1$-$C_{30}$ ou un radical aralkyle,
- u, v, w et x sont chacun indépendamment des nombres entiers allant de 0 à 900,

sous réserve que la somme v+w+x soit différente de 0 et que la somme u+v+w+x va de 1 à 900.

**39.** Produit selon l'une des revendications 28 à 38, **caractérisé en ce que** la phase liquide non volatile de la seconde composition contient une phase siliconée comprenant une huile siliconée phénylée de basse viscosité et une huile siliconée phénylée de haute viscosité.

**40.** Produit selon la revendication précédente, **caractérisé en ce que** l'huile siliconée phénylée de basse viscosité présente une viscosité à 25°C allant de 5 à 499 cSt et l'huile siliconée phénylée de haute viscosité présente une viscosité à 25°C allant de 500 à 10 000 cSt.

**41.** Produit selon la revendication 39 ou 40, **caractérisé en ce que** l'huile siliconée phénylée de basse viscosité présente une viscosité allant de 5 à 300 cSt à 25°Ct et l'huile siliconée phénylée de haute viscosité présente une viscosité allant de 600 à 5000 cSt à 25°C.

**42.** Produit selon l'une des revendications 39 à 41, **caractérisé en ce que** l'huile siliconée phénylée de basse viscosité satisfait à la formule (III) suivante :

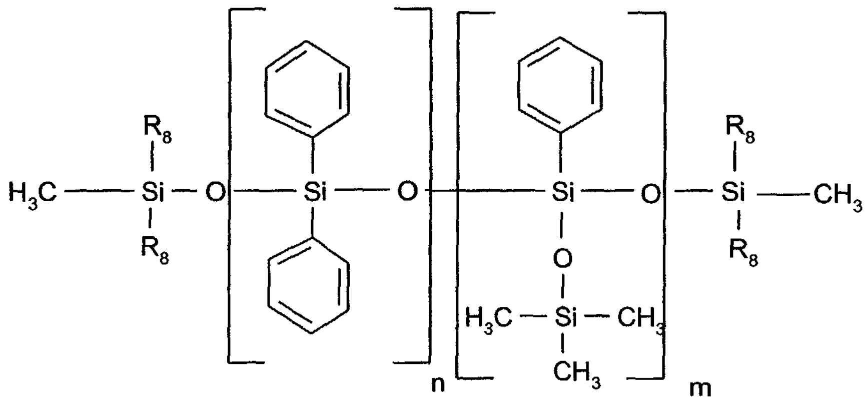

(III)

dans laquelle :

- . $R_8$ est un radical alkyle en $C_1$-$C_{30}$, un radical aryle, ou un radical aralkyle,
- . n est un nombre entier allant de 0 à 100, et mieux, inférieur à 100,
- . m est un nombre entier allant de 0 à 100, sous réserve que la somme m+n va de 1 à 100 , et mieux, est inférieure à 100.

**43.** Produit selon l'une des revendications 39 à 42, **caractérisé en ce que** le rapport en poids entre l'huile siliconée phénylée de basse viscosité et l'huile siliconée phénylée de haute viscosité peut aller par exemple de 70/30 à 30/70, mieux de 60/40 à 40/60 et encore mieux de 55/45 à 45/55.

**44.** Produit selon l'une des revendications 28 à 43, **caractérisé en ce que** la phase liquide non volatile de la seconde composition représente de 1 à 100%, de préférence de 5 à 95%, mieux de 20 à 80% et mieux encore, de 40 à 80 % du poids total de la seconde composition.

**45.** Produit selon l'une des revendications précédentes, **caractérisé en ce que** le milieu physiologiquement acceptable de la première et/ou de la seconde composition contient un ou plusieurs actifs cosmétiques ou dermatologiques.

**46.** Produit selon l'une des revendications précédentes, **caractérisé en ce que** le milieu physiologiquement acceptable de la première et/ou de la seconde composition contient des corps gras additionnels choisis parmi les cires, les huiles, les gommes et les corps gras pâteux, hydrocarbonés, siliconés et/ou fluorés, d'origine végétale, animale, minérale ou de synthèse et leurs mélanges.

**47.** Produit selon l'une des revendications précédentes, **caractérisé en ce que** le milieu physiologiquement acceptable de la première composition contient une gomme.

**48.** Produit selon la revendication 47, **caractérisé en ce que** la gomme est une gomme de silicone.

**49.** Produit selon l'une des revendications précédentes, **caractérisé en ce que** le milieu physiologiquement acceptable de la seconde composition contient un corps gras pâteux et/ou une cire.

**50.** Produit selon l'une des revendications précédentes, **caractérisé en ce que** le milieu physiologiquement acceptable de la première et/ou de la seconde composition contient en outre au moins un additif choisi parmi les épaississants d'huile ou de phase aqueuse, les antioxydants, les parfums, les conservateurs, les tensioactifs, les polymères liposolubles et leurs mélanges.

**51.** Produit selon l'une des revendications précédentes, **caractérisé en ce que** la première et/ou la seconde composition est sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules ou d'une poudre.

**52.** Produit selon l'une des revendications précédentes, **caractérisé en ce que** la première ou la seconde composition,

ou les deux, sont sous forme anhydre.

**53.** Produit selon l'une des revendications précédentes, **caractérisé en ce qu'**il se présente sous la forme d'un fond de teint, d'un fard à joues ou à paupières, d'un rouge à lèvres, d'un produit ayant des propriétés de soin, d'un eye-liner, d'un produit anti-cernes, ou d'un produit de maquillage du corps.

**54.** Produit selon l'une des revendications précédentes, **caractérisé en ce qu'**il se présente sous la forme d'un rouge à lèvres.

**55.** Produit selon l'une des revendications précédentes, **caractérisé en ce que** la première et/ou la seconde composition est sous forme solide.

**56.** Procédé de maquillage de la peau et/ou des lèvres et/ou des phanères consistant à appliquer sur la peau, les lèvres et/ou les phanères une première couche d'une première composition comprenant, dans un premier milieu physiologiquement acceptable des particules de polymère dispersées et stabilisées en surface par un stabilisant dans une phase organique liquide et un agent de coloration, puis à appliquer, sur tout ou partie de ladite première couche, une seconde couche d'une seconde composition comprenant un second milieu physiologiquement acceptable.

**57.** Procédé de maquillage de la peau et/ou des lèvres et/ou des phanères consistant à appliquer sur la peau, les lèvres et/ou les phanères une première couche d'une première composition comprenant, dans un premier milieu physiologiquement acceptable, des particules de polymère dispersées et stabilisées en surface par un stabilisant dans une phase organique liquide et un agent de coloration, à laisser sécher ladite première couche, puis à appliquer, sur tout ou partie de la première couche, une seconde couche d'une seconde composition comprenant un second milieu physiologiquement acceptable.

**58.** Procédé selon la revendication 56 ou 57, **caractérisé en ce que** la phase organique liquide de la première composition est conforme à la revendication 11 ou 12.

**59.** Procédé selon l'une des revendications 56 à 58, **caractérisé en ce que** le milieu physiologiquement acceptable de la seconde composition contient une phase liquide non volatile à température ambiante et pression atmosphérique.

**60.** Procédé selon la revendication 59, **caractérisé en ce que** la phase liquide non volatile de la seconde composition est conforme à l'une quelconque des revendications 28 à 44.

**61.** Procédé selon l'une des revendications précédentes, **caractérisé en ce que** la prémière composition est sous forme solide.

**62.** Procédé de maquillage de la peau et/ou des lèvres et/ou des phanères consistant à appliquer sur la peau et/ou les lèvres et/ou les phanères un produit cosmétique de maquillage selon l'une des revendications 1 à 55.

**63.** Kit de maquillage comprenant un produit selon l'une des revendications 1 à 55.

**64.** Kit selon la revendication 63, **caractérisé en ce qu'**il contient des moyens d'application de la première et/ou de la seconde compositions sur la peau, les lèvres et/ou les phanères.

**65.** Kit selon la revendication 63 ou 64, **caractérisé en ce qu'**il contient des moyens d'application choisis parmi les pinceaux, brosses, stylos, crayons, feutres, plumes, éponges, mousses.

**66.** Kit selon la revendication 64 ou 65, **caractérisé en ce** les moyens d'application sont des feutres.

**67.** Kit selon l'une des revendications 63 à 66, **caractérisé en ce que** les première et seconde compositions sont conditionnées dans des compartiments ou récipients distincts.

**68.** Composition cosmétique comprenant, dans un milieu physiologiquement acceptable, des particules de polymère dispersées et stabilisées en surface par un stabilisant dans une phase organique liquide, un agent de coloration et un agent rhéologique choisi parmi les copolymères d'oléfines à cristallisation controlée et leurs mélanges.

**69.** Composition selon la revendication précédente, **caractérisée en ce** l'agent rhéologique est un copolymère éthylène/octène.

**70.** Utilisation cosmétique du produit selon l'une des revendications 1 à 55 pour améliorer les propriétés de confort, de brillance, de non transfert et/ou de non migration et/ou de tenue du maquillage sur la peau, les lèvres et/ou les phanères.

**71.** Utilisation d'un produit cosmétique de maquillage contenant une première et une seconde compositions, la première composition comprenant, dans un premier milieu physiologiquement acceptable, des particules de polymère dispersées et stabilisées en surface par un stabilisant dans une phase organique liquide et un agent de coloration, et la seconde composition comprenant un second milieu physiologiquement acceptable, pour conférer à la peau et/ou les lèvres et/ou les phanères un maquillage confortable, brillant, non transfert et/ou non migrant et/ou de bonne tenue.

**72.** Support maquillé comprenant une première couche d'une première composition comprenant, dans un premier milieu physiologiquement acceptable, des particules de polymère dispersées et stabilisées en surface par un stabilisant dans une phase organique liquide et un agent de coloration, et une seconde couche d'une seconde composition, déposée sur tout ou partie de la première couche, comprenant un second milieu physiologiquement acceptable.

**73.** Support selon la revendication 72, **caractérisé en ce qu'**il se présente sous la forme de faux ongles, faux cils, perruques.

Office européen des brevets

# RAPPORT DE RECHERCHE EUROPEENNE

Numéro de la demande
EP 02 29 0816

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| P,X | EP 1 142 551 A (SHISEIDO CO., LTD) 10 octobre 2001 (2001-10-10) * le document en entier * --- | 1 | A61K7/02 A61K7/00 A41G3/00 A41G5/00 A63H3/44 |
| A | EP 0 908 175 A (L'OREAL) 14 avril 1999 (1999-04-14) * revendication 1; exemple 4 * --- | 1 | |
| A | EP 1 036 554 A (L'OREAL) 20 septembre 2000 (2000-09-20) * le document en entier * --- | 1 | |
| A,P | DATABASE CHEMICAL ABSTRACTS 'en ligne! retrieved from STN Database accession no. 135:50907 XP002201512 * abrégé * & JP 2001 169823 A (MAKIYAJU K.K.) 26 juin 2001 (2001-06-26) --- | 1,72,73 | |
| A | DATABASE CHEMICAL ABSTRACTS 'en ligne! retrieved from STN Database accession no. 133:325508 XP002201513 * abrégé * & JP 2000 308516 A (GENERAL CO., LTD) 7 novembre 2000 (2000-11-07) ----- | 1,72,73 | DOMAINES TECHNIQUES RECHERCHES (Int.Cl.7): A61K |

Le présent rapport a été établi pour toutes les revendications

| Lieu de la recherche | Date d'achèvement de la recherche | Examinateur |
|---|---|---|
| LA HAYE | 13 juin 2002 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 02 29 0816

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-06-2002

| Document brevet cité au rapport de recherche | | Date de publication | Membre(s) de la famille de brevet(s) | | | Date de publication |
|---|---|---|---|---|---|---|
| EP 1142551 | A | 10-10-2001 | JP | 2001131024 | A | 15-05-2001 |
| | | | EP | 1142551 | A1 | 10-10-2001 |
| | | | WO | 0134101 | A1 | 17-05-2001 |
| EP 908175 | A | 14-04-1999 | FR | 2768926 | A1 | 02-04-1999 |
| | | | BR | 9804138 | A | 14-12-1999 |
| | | | CA | 2245989 | A1 | 01-04-1999 |
| | | | EP | 0908175 | A1 | 14-04-1999 |
| | | | JP | 11158030 | A | 15-06-1999 |
| | | | PL | 328909 | A1 | 12-04-1999 |
| | | | US | 6258345 | B1 | 10-07-2001 |
| | | | US | 2001026811 | A1 | 04-10-2001 |
| EP 1036554 | A | 20-09-2000 | FR | 2790665 | A1 | 15-09-2000 |
| | | | BR | 0000810 | A | 23-01-2001 |
| | | | EP | 1036554 | A1 | 20-09-2000 |
| | | | JP | 2000256134 | A | 19-09-2000 |
| JP 2001169823 | A | 26-06-2001 | AUCUN | | | |
| JP 2000308516 | A | 07-11-2000 | AUCUN | | | |

EPO FORM P0460

Pour tout renseignement concernant cette annexe : voir Journal Officiel de l'Office européen des brevets, No.12/82